# FASCICULE DE BREVET EUROPEEN

(11) **EP 3 692 913 B1**
(45) Date de publication et mention de la délivrance du brevet: **30.11.2022**
(21) Numéro de dépôt: 20155852.5
(22) Date de dépôt: 06.02.2020
(51) Int. Cl.: A61B 5/107, A41H 1/02, G01B 3/10

(54) **DISPOSITIF DE MESURE DE LA CIRCONFÉRENCE D'UN OBJET, EN PARTICULIER D'UN MEMBRE CORPOREL**
VORRICHTUNG ZUM MESSEN DES UMFANGS EINES OBJEKTS, INSBESONDERE EINES KÖRPERGLIEDS
DEVICE FOR MEASURING THE CIRCUMFERENCE OF AN OBJECT, IN PARTICULAR OF A LIMB

(30) Priorité: 07.02.2019 BE 201905075
(43) Date de publication de la demande: 12.08.2020
(73) Titulaire: Just A New Health, 1320 Beauvechain (BE)
(72) Inventeur: Harfouche, Joseph, 1190 Forest (BE)
(74) Mandataire: Gevers Patents

(56) Documents cités:
- WO-A1-2014/191513
- WO-A1-2018/091462
- FR-A1- 3 065 625
- GB-A- 2 452 256
- GB-A- 2 500 627
- US-B1- 8 196 308

## Description

La présente invention se rapporte à un dispositif de mesure de la circonférence d'un objet, en particulier à un dispositif de mesure de la circonférence d'un membre corporel.

Une mesure de la circonférence de membres corporels tels qu'un bras ou une jambe est notamment recommandée dans le cadre du bilan et du suivi du traitement de kinésithérapie, par exemple pour le traitement des lymphoedèmes, c'est-à-dire de gonflements d'une partie du corps suite à une accumulation de liquide lymphatique dans les tissus interstitiels. De tels gonflements apparaissent lorsque les lymphangions qui constituent les vaisseaux lymphatiques sont dégradés ou non-fonctionnels (lymphoedème primaire) ou lorsque les vaisseaux lymphatiques eux-mêmes sont endommagés ou obstrués et lorsque des nodules lymphatiques ont été enlevés (lymphoedème secondaire).

Plus particulièrement, les lymphoedèmes secondaires sont une conséquence d'un dommage ou d'un traumatisme par exemple causé par un accident, une chirurgie, une infection grave ou encore une radiothérapie ou d'autres causes.

Ces gonflements concernent essentiellement les membres supérieurs et inférieurs tels que par exemple les bras, les pieds, les jambes, les cuisses et les mains mais peuvent aussi se produire au niveau d'autres parties du corps comme le cou, l'abdomen, le dos ou encore les seins. A noter que le lymphoedème secondaire des membres supérieurs est principalement causé par le traitement chirurgical du creux axillaire du cancer du sein consistant en une ablation des ganglions de l'aisselle.

Afin de déterminer dans quelle mesure un lymphoedème (primaire ou secondaire) doit être traité, il convient d'en suivre l'évolution. A titre d'exemple, si on considère le lymphoedème secondaire des membres supérieurs, le guide de pratique clinique pour la prise en charge et le traitement du cancer du sein (Guides de pratique clinique pour la prise en charge et le traitement du cancer du sein, Canadian Medical Association Journal) préconise de mesurer la circonférence branchiale à quatre points : aux articulations métacarpiennes-phalangiennes, aux poignets, à 10 cm en aval et à 15 cm en amont des épicondyles latéraux (coude). Il est considéré qu'une différence de plus de 2 cm de la circonférence, entre deux mesures à l'un de ces quatre points de mesure, justifie le traitement du lymphoedème. Une différence de plus de 2 cm de la circonférence entre un membre corporel (par exemple le bras droit) présentant un lymphoedème et un membre corporel correspondant (par exemple le bras gauche) ne présentant pas de lymphoedème indique également qu'il convient de procéder à un traitement de ce gonflement.

Il est donc nécessaire de disposer d'un dispositif ou d'un instrument de mesure qui permette de mesurer la circonférence de membres corporels ponctuellement et à un même endroit afin d'être en mesure de décider si un traitement du lymphoedème est d'application ou non. Il convient particulièrement de disposer d'un outil de mesure qui permette une mesure précise et fiable puisque la marge d'erreur doit être faible et seulement de l'ordre de quelques millimètres, de préférence de l'ordre de moins de 5 millimètres et plus préférentiellement de l'ordre de moins de 2 millimètres.

Une mesure de la circonférence de membres corporels est également indiquée pour constater une diminution du volume des muscles squelettiques et en suivre l'évolution. Une telle diminution du volume musculaire (ou perte de la masse musculaire) peut par exemple être due à une amyotrophie (atrophie et/ou disparition de la fibre musculaire striée), à une sarcopénie (syndrome gériatrique) ou encore à des myopathies (maladie neuro-musculaires). Ces pathologies nécessitent un suivi et notamment un traitement de kinésithérapie au cours duquel une mesure précise de la circonférence du membre corporel comprenant le muscle considéré est indispensable. Ici aussi, l'évolution de la circonférence du membre corporel peut être suivie par comparaison de deux mesures effectuées à un même endroit après une période de temps prédéterminée ou en comparant les circonférences d'un membre corporel « sain » et d'un membre corporel correspondant présentant une diminution du volume musculaire (perte de masse musculaire).

Une mesure de la circonférence de membres corporels est également indiquée pour constater une diminution du volume lors de suivis d'un régime diététique et lors de traitements anti-cellulites lors desquels un affinement du membre est attendu.

Une mesure de la circonférence de membres corporels est également indiquée pour constater une modification du volume lors d'un entrainement dans le cadre d'un programme de musculation où une augmentation du volume musculaire est attendue ou espérée.

Il est bien entendu que tout autre état ou pathologie entrainant une variation de la circonférence au niveau d'un membre corporel fait partie du cadre de la présente invention.

Par ailleurs, en Europe, plusieurs pays légifèrent à ce sujet, comme par exemple les Directives de l'Institut National d'Assurance Maladie-Invalidité (INAMI) qui recommandent aux praticiens (médecins, kinésithérapeutes, ...) d'effectuer des mesures tous les 4 cm le long du membre corporel présentant un gonflement ou souffrant d'une diminution du volume musculaire. Un nombre de mesures conséquent doit donc être effectué et il convient ainsi de disposer d'un outil de mesure qui, une fois positionné le long d'un membre corporel permette de relever ou d'effectuer des mesures à intervalles réguliers sur ce membre corporel de façon fiable, rapide, précise et reproductible.

Des dispositifs de mesure de la circonférence d'un objet et plus particulièrement de la circonférence d'un membre corporel sont connus des documents WO2014/191513 et WO2018/091462.

Notons que les documents GB2452256, FR3065625 et GB2500627 divulguent une solution pour attacher des mètres-rubans les uns aux autres et que le document US8196308 divulgue une solution pour attacher des moyens de marquage à une paroi d'un enrouleur pour mètre-ruban.

Malheureusement, même si les dispositifs selon ces documents antérieurs permettent bien de réaliser des mesures itératives le long de membres corporels, les coulisseaux prévus pour coulisser le long d'une coulisse et qui sont prévus pour être reliés à des éléments de mesure agencés pour entourer le membre corporel lors de la mesure de sa circonférence, peuvent être la source de légères erreurs de mesure dès lors qu'ils peuvent effectuer un déplacement non souhaité au moment même où une mesure précise de circonférence doit être effectuée. Par exemple, au moment même où un opérateur opère une mesure de circonférence se devant d'être très précise à l'endroit précis où la mesure doit être réalisée, un mouvement involontaire du patient et/ou un mouvement involontaire de l'opérateur peut donner lieu à un déplacement par coulissement (glissement) du coulisseau le long de la coulisse, ceci impliquant que la mesure n'est pas effectuée au bon endroit où elle est justement censée être prise. Ceci est bien entendu problématique puisque, comme indiqué plus haut, seule une marge d'erreur de l'ordre de quelques millimètres peut être tolérée pour chaque mesure effectuée. Or, chaque déplacement du coulisseau, même uniquement de quelques millimètres, peut se traduire par une erreur de mesure de plusieurs centimètres de la circonférence.

L'invention a pour but de pallier les inconvénients de l'état de la technique en procurant un dispositif de mesure de la circonférence de membres corporels qui permette de réaliser des mesures rapides, simples, précises, fiables et reproductibles quel que soit le membre corporel considéré, que ce dernier soit court ou long, présente des courbures et/ou des protubérances ou non et à n'importe quel endroit le long du membre corporel considéré. En outre l'invention a également pour objectif de procurer un dispositif de mesure qui présente une taille raisonnable, qui soit maniable, léger et peu encombrant, ce qui constitue un avantage certain dans le domaine médical où les professionnels du domaine de la santé, se déplacent de chambres en chambres ou d'un cabinet à l'autre ou d'une salle de sport à l'autre pour voir leurs patients ou leurs clients.

Pour résoudre au moins en partie les problèmes de l'état de la technique, il est prévu, suivant l'invention, un dispositif de mesure de la circonférence d'un objet, en particulier d'un membre corporel comprenant :
- un élément longitudinal souple gradué ou non présentant une première direction longitudinale, agencé pour être apposé le long dudit objet, en particulier le long dudit membre corporel, ledit élément longitudinal souple étant relié à une sangle distale détachable ou non et/ou à une sangle proximale détachable ou non inscrite chacune dans un plan sensiblement perpendiculaire à ladite direction longitudinale, ledit élément longitudinal souple comprenant différentes zones de connexion, et
- un système de mesure comprenant un organe de connexion permettant d'assurer une connexion dudit système de mesure à au moins une des différentes zones de connexion dudit élément longitudinal souple, ledit système de mesure étant sous la forme d'un enrouleur d'un élément de mesure souple gradué ou non, agencé pour être apposé et former une boucle autour dudit objet, en particulier autour dudit membre corporel, lorsque ledit élément de mesure est en position de mesure, ledit système de mesure comprenant :
   - au moins une paroi et un orifice de sortie agencé pour permettre une sortie d'au moins une partie déroulée dudit élément de mesure, ladite au moins une partie déroulée dudit élément de mesure étant définie entre ledit orifice de sortie et une extrémité distale dudit élément de mesure, ladite extrémité distale dudit élément de mesure étant munie d'un élément de connexion, et
   - un moyen de connexion dudit l'élément de connexion de ladite extrémité distale dudit élément de mesure,

ledit organe de connexion, permettant d'assurer une connexion dudit système de mesure sous la forme d'un enrouleur à au moins une des différentes zones de connexion dudit élément longitudinal souple,
ledit dispositif de mesure étant caractérisé en ce que lesdites différentes zones de connexion (2, 2', 2", ...) dudit élément longitudinal souple (1) sont des zones fixes de connexion, et en ce que ledit organe de connexion (4) est situé sur une paroi dudit système de mesure (3) entre ledit orifice de sortie (6) et ledit moyen de connexion (8) dudit élément de connexion (7) de ladite extrémité distale dudit élément de mesure (5).

De préférence, après formation d'une boucle autour du membre corporel dont la circonférence doit être mesurée, ledit organe de connexion est situé à l'intérieur de la boucle. Eventuellement, plutôt que d'être situé à l'intérieur même de la boucle, ledit organe de connexion peut être localisé en une zone adjacente à l'orifice de sortie et/ou audit moyen de connexion dudit élément de connexion de ladite extrémité distale dudit élément de mesure.

Un tel dispositif tel que préconisé suivant l'invention est particulièrement avantageux et permet de mesurer la circonférence de n'importe quel membre corporel à n'importe quel endroit sur ce membre, de façon rapide, précise, reproductible et fiable, que ce membre corporel présente des courbures et/ou des protubérances ou non, ceci tout en assurant que la mesure effectuée le sera bel et bien à l'endroit prévu une fois l'élément longitudinal et le système de mesure positionnés adéquatement.

En effet, selon la présente invention, une mesure simple, précise et rapide de la circonférence d'un membre corporel peut être obtenue en toute circonstance puisque l'élément longitudinal souple comprend des zones fixes de connexion et que le système de mesure sous la forme d'un enrouleur comprend un organe de connexion permettant d'assurer sa connexion à au moins une zone fixe de connexion de l'élément longitudinal. Au sens de la présente invention, une « zone fixe de connexion » est une zone (un endroit) sur l'élément longitudinal qui est définie par un positionnement précis, permanent et non déplaçable. A titre d'exemple, une telle « zone fixe de connexion » peut être une perforation (organe femelle agencé pour coopérer avec un organe mâle) pratiquée dans/au travers de l'élément longitudinal. Au sens de l'invention, une telle « zone fixe de connexion » peut également être un tenon (organe mâle agencé pour coopérer avec un organe femelle). Par conséquent, une fois que le système de mesure est connecté à l'élément longitudinal par l'intermédiaire de l'organe de connexion, il se trouve connecté à un endroit (en une zone) précis de l'élément longitudinal, endroit (zone) dont il ne peut pas bouger avant qu'une déconnexion ne soit effectuée par un opérateur, c'est-à-dire avant que l'organe de connexion ne soit déconnecté de la zone fixe de connexion à laquelle il a été préalablement connecté. Ceci implique que, même si le patient et/ou l'opérateur effectue un mouvement involontaire lors de la prise de mesure de la circonférence, le système de mesure reste en place sur l'élément longitudinal, ceci à l'endroit précis où la mesure doit être effectuée.

De plus, selon l'invention, l'organe de connexion étant situé sur une paroi du système de mesure entre l'orifice de sortie et le moyen de connexion dudit élément de connexion de ladite extrémité distale dudit élément de mesure, il est particulièrement aisé d'apposer l'élément de mesure sous forme d'une boucle autour du membre corporel dont la circonférence doit être mesurée. En effet, un tel positionnement de l'organe de connexion fait que ce dernier est situé à l'intérieur de la boucle lorsque celle-ci est formée autour de l'objet (du membre corporel) dont la circonférence doit être mesurée. Le fait que l'organe de connexion soit situé à l'intérieur de la boucle fait qu'il ne gène ni la formation de cette dernière ni son positionnement en contact étroit avec le membre dont la circonférence doit être mesurée. Ce positionnement de l'organe de connexion tel que prévu selon l'invention est tel que l'élément de mesure n'est pas en contact avec l'organe de connexion, lequel ne fausse alors pas la prise de mesure de la circonférence comme ce serait le cas si l'élément de mesure devait passer sur l'organe de connexion afin de pouvoir être connecté au niveau du moyen de connexion de l'élément de connexion de l'extrémité distale de l'élément de mesure.

Ainsi, un tel positionnement de l'organe de connexion n'entrave pas la formation d'une boucle autour du membre corporel, permet à l'opérateur de manipuler l'élément de mesure avec facilité et permet de garantir une précision des mesures effectuées. Une fois l'organe de connexion en connexion avec une zone fixe de connexion de l'élément longitudinal, l'élément de mesure est libre d'être déroulé sans exercer de traction sur l'élément longitudinal et sans risquer que la mesure ne soit effectuée à un mauvais endroit puisque l'organe de connexion assure que le système de mesure (l'enrouleur) reste en place, ceci peu importe les manipulations effectuées avec l'élément de mesure afin de l'enrouler autour du membre corporel. Il apparait donc que, au contraire d'un dispositif tel qu'illustré à la figure 5 du document WO2018/091462 où l'élément de mesure est connecté à un coulisseau, la partie déroulée de l'élément de mesure est totalement libre avec un dispositif selon la présente l'invention.

En outre, comme l'élément longitudinal est formé dans un matériau souple, il peut parfaitement, sur l'ensemble de sa longueur, être apposé le long de membres corporels, directement contre la peau, même si ces membres corporels présentent des protubérances et/ou des creux.

Par ailleurs, puisque l'élément longitudinal est formé et fabriqué dans un matériau souple, il peut être facilement enroulé et/ou plié, ce qui permet d'en minimiser la taille et en facilite le rangement, le transport et la manipulation.

Par les termes « souple », ou « formé dans un matériau souple », on entend, au sens de la présente invention, un élément dont la souplesse lui permet de suivre/entourer, étroitement et par contact, la surface de l'objet ou du membre corporel dont la circonférence doit être mesurée, cette souplesse permettant par ailleurs un pliage, une courbure ou un enroulage.

Selon la présente invention, des mesures précises, simples, fiables et reproductibles de la circonférence de membres corporels peuvent donc être réalisées dans toute circonstance, quel que soit le membre corporel considéré ou l'endroit du membre corporel considéré puisque le dispositif de mesure suivant l'invention permet d'assurer que la mesure de circonférence sera bien effectuée à l'endroit précis envisagé sans qu'un déplacement du système de mesure et donc de l'élément de mesure ne puisse fausser les relevés de mesure effectués. Comme indiqué plus haut, de telles mesures à la fois précises, simples, fiables et reproductibles de la circonférence de membres corporels sont indispensables afin d'éliminer toute marge d'erreur qui pourrait fausser les diagnostics et remettre en cause le principe même de la mesure de la circonférence de membres corporels comme outil décisionnel avant d'entamer ou non un traitement.

De préférence, selon l'invention, l'organe de connexion du système de mesure et/ou les différentes zones fixes de connexion de l'élément longitudinal est/sont configuré(s) de telle sorte qu'il(s) assure(nt) une inscription et un maintien dudit élément de mesure dans un plan perpendiculaire à la direction longitudinale de l'élément longitudinal.

Avantageusement, selon l'invention, lesdites différentes zones fixes de connexion sont disposées à intervalles réguliers le long dudit élément longitudinal.

Selon l'invention, ledit organe de connexion dudit système de mesure est un organe de type mâle ou femelle agencé pour coopérer et assurer une connexion avec au moins une desdites zones fixes de connexion dudit élément longitudinal étant une zone de type mâle ou femelle correspondante.

Avantageusement, selon l'invention, le moyen de connexion de l'élément de connexion de l'extrémité distale de l'élément de mesure est situé à une distance d dudit orifice de sortie de l'enrouleur, ladite distance d définie entre ledit orifice de sortie et ledit moyen de connexion étant inférieure ou égale à 4 cm, de préférence inférieure ou égale à 3 cm, préférentiellement inférieure ou égale à 2 cm, plus préférentiellement inférieure ou égale à 1 cm, de façon préférée égale à zéro. Selon un mode de réalisation préféré, la distance d est égale à zéro ce qui signifie que le moyen de connexion est accolé et est donc en contact avec l'orifice de sortie de l'enrouleur. Un tel positionnement relatif entre le moyen de connexion et l'orifice de sortie de l'enrouleur permet d'autant plus d'éviter toute erreur et tout biais lors de la prise de mesure d'une circonférence d'un objet/d'un membre corporel et de mesurer n'importe quelle circonférence aussi petite soit-elle (par exemple la circonférence d'un doigt). Par exemple, l'orifice de sortie étant délimité par quatre parois, il est prévu suivant l'invention que le moyen de connexion soit placé en contact direct de l'une de ces parois de telle sorte que la distance d soit égale à zéro ou du moins seulement de l'ordre de quelques millimètres.

De préférence, selon l'invention, ledit moyen de connexion et/ou ledit élément de connexion sont connectés par l'intermédiaire d'un aimant situé au niveau dudit moyen de connexion ou au niveau dudit élément de connexion.

De préférence, selon l'invention, ledit élément longitudinal comprend des capteurs permettant de déterminer et de définir un positionnement dudit système de mesure sur l'élément longitudinal. En particulier, ces capteurs sont agencés pour qu'une information quant au positionnement dudit système de mesure sur l'élément longitudinal puisse être transmise à l'opérateur ; par exemple par affichage de ce positionnement au niveau du système de mesure et/ou par affichage de ce positionnement sur un dispositif externe (par exemple un smartphone), les capteurs, le système de mesure et/ou le dispositif externe interagissant, par exemple par l'intermédiaire d'un module électronique, de telle sorte qu'une information relative à ce positionnement soit traitée et affichée.

De préférence, selon l'invention, la boucle formée autour dudit objet, en particulier autour dudit membre corporel, est inscrite dans un seul et unique plan passant par un axe longitudinal médian défini le long dudit élément de mesure. Un tel positionnement de la boucle et donc de la partie déroulée de l'élément de mesure selon l'invention permet d'assurer une précision optimale de la mesure de la circonférence. En effet, tout biais est minimisé dès lors que la boucle est positionnée dans un seul et unique plan.

Avantageusement, selon l'invention, le système de mesure est un enrouleur à ressort éventuellement muni d'un système de blocage et/ou d'un système de rappel dudit élément de mesure. Le système de blocage permet avantageusement, après déroulement d'au moins une partie de l'élément de mesure, de bloquer cette dernière afin de faciliter la mise en place de l'élément de mesure autour de l'objet/membre corporel. Par ailleurs, suite à la mise en place de l'élément de mesure autour de l'objet/membre corporel et suite à la connexion de la partie distale de l'élément de mesure au moyen de connexion, il est prévu qu'un système de rappel de l'élément de mesure permette un placement automatique et adéquat de l'élément de mesure autour de l'objet/membre corporel dont la circonférence doit être mesurée. En effet, puisque la partie déroulée de l'élément de mesure est généralement plus longue que la circonférence à mesurer réellement, ceci pour des raisons de facilité de mise en place du dispositif de mesure, il convient ensuite de faire en sorte que l'élément de mesure épouse parfaitement le contour de l'objet/membre corporel, ceci pouvant être assuré selon l'invention par un système de rappel (automatique) de l'élément de mesure, en particulier de la partie déroulée de l'élément de mesure. Bien entendu, tout type d'enrouleur adéquat entre dans le cadre de la présente invention.

Préférentiellement, selon l'invention, ledit système de mesure comprend un système de rappel dudit élément de mesure, par exemple un ressort ou un moteur, exerçant sur ledit élément de mesure une tension liée à un poids compris entre 5 et 5000 g, préférentiellement entre 5 et 3000 g, préférentiellement entre 15 et 1500 g, plus préférentiellement encore entre 15 et 1000 g, avantageusement entre 15 et 500 g, plus avantageusement entre 15 et 150 g.

Plus préférentiellement, selon l'invention, ledit système de rappel dudit élément de mesure, par exemple un ressort ou un moteur, est un système de rappel qui exerce sur ledit élément de mesure une tension liée à un poids compris entre 5 et 80 g, de préférence entre 5 et 60 g, de préférence entre 70 et 80 g. Plus préférentiellement encore, ledit système de rappel dudit élément de mesure, par exemple un ressort ou un moteur, est un système de rappel qui exerce sur ledit élément de mesure une tension liée à un poids supérieur ou égal à 50 g et inférieur à ou égal à 5000 g, préférentiellement supérieur ou égal à 70 g et inférieur à ou égal à 5000 g.

La tension à exercer sur l'élément de mesure sera prédéterminée selon l'application visée par la mesure de la circonférence pour permettre d'effectuer des mesures reproductibles selon cette application (œdème lymphatique, œdème traumatique, fabrication de bas de contention, ...).

Avantageusement, le dispositif de mesure selon l'invention, comprend un capteur de tension, de préférence un capteur de tension logé dans l'enrouleur, pour détecter que l'élément de mesure est tendu afin de pouvoir mesurer la tension.

De préférence, selon l'invention, l'orifice de sortie dudit enrouleur présente une section similaire à la section dudit élément de mesure. De cette façon, l'orifice de sortie guide véritablement l'élément de mesure de telle sorte que ce dernier s'appose correctement autour de l'objet/membre corporel dont la circonférence est mesurée. En particulier, le fait que ces deux sections soient similaires permet que la boucle formée autour de l'objet/membre corporel soit inscrite d'autant mieux dans un seul et unique plan passant par un axe longitudinal médian défini sur l'élément de mesure. Au sens de l'invention, plus la largeur de l'élément de mesure est réduite, plus la prise de mesure est fiable, précise et reproductible, ceci notamment en minimisant le bâillement de l'élément de mesure observé au niveau de la face antérieure de l'avant-bras, au niveau de la jambe, au niveau du mollet ou encore au niveau de tout autre membre dont la forme n'est pas forcément cylindrique.

Préférentiellement, selon l'invention, ladite extrémité distale dudit élément de mesure munie d'un élément de connexion coopère avec ledit moyen de connexion de telle sorte à assurer une connexion entre ledit moyen de connexion et ladite extrémité distale dudit élément de mesure. Une telle connexion peut être réalisée par l'intermédiaire d'un système mâle-femelle permettant par exemple un emboitement de l'élément de connexion présent au niveau de la partie distale de l'élément de mesure dans le moyen de connexion. Il est évident que tout autre type de système ou dispositif pouvant assurer une telle connexion fait partie intégrante de la présente invention, comme par exemple une fixation à l'aide d'un aimant ou une fixation à l'aide de Velcro^{®}.

De préférence, selon l'invention, ledit enrouleur présente une fenêtre de lecture d'une circonférence d'un objet, en particulier d'une circonférence d'un membre corporel.

Avantageusement, le dispositif de mesure selon l'invention, comprend en outre :
- un module électronique de mesure apte à déterminer une mesure de circonférence à partir d'un déroulement dudit élément de mesure ; et
- des moyens d'affichages pour afficher une mesure de circonférence déterminée par ledit module électronique.

Ce mode de réalisation présente l'avantage de pouvoir effectuer une mesure rapidement : les moyens d'affichage permettent à l'opérateur de réaliser directement une lecture d'une mesure de circonférence. L'utilisation d'un module électronique apte à déterminer une mesure de circonférence permet par ailleurs une meilleure reproductibilité de la mesure de la circonférence d'un objet, en particulier de la circonférence d'un membre corporel, puisque les variations dues à l'intervention d'un opérateur sont fortement limitées de par l'automatisation/digitalisation du dispositif de mesure. La lecture de la mesure est par ailleurs plus aisée car la valeur de la circonférence est affichée grâce aux moyens d'affichages.

De préférence, le dispositif de mesure selon l'invention comprend en outre des moyens de communication pour communiquer à un autre dispositif électronique (par exemple un smartphone) une mesure de circonférence déterminée par ledit module électronique. Cette communication, par exemple par Bluetooth, du dispositif de mesure avec un autre dispositif électronique grâce aux moyens de communication permet avantageusement d'éviter par exemple la retranscription par la personne qui effectue la mesure de circonférence de la mesure affichée par les moyens d'affichages.

De préférence, le dispositif de mesure selon l'invention comprend en outre des moyens de contrôle pour commander une transmission (par exemple par transmission Bluetooth) d'une circonférence déterminée par ledit module électronique par les moyens de communication. L'avantage de disposer de moyens de contrôle compris dans le dispositif de mesure est que l'opérateur peut déterminer le moment où il souhaite que la mesure de circonférence soit communiquée, de préférence aux moyens d'affichage (par exemple pour un affichage digital) et de préférence aux moyens de communication afin que la mesure soit communiquée à un autre dispositif électronique, par exemple à un smartphone.

De manière avantageuse, le dispositif selon l'invention comprend ledit capteur de tension et/ou un moteur, dans lequel une force de tension peut être programmée et un signal (par exemple un signal sonore) averti l'utilisateur lorsque cette force de tension prédéterminée est atteinte. Ce dernier permettra de serrer suffisamment l'élément de mesure autour de l'objet/membre corporel dont la circonférence doit être mesurée ou examinée. Ceci est particulièrement avantageux pour les praticiens tels que les bandagistes qui devraient être en mesure de pouvoir appliquer une tension suffisante pour réaliser une prise de mesure ou un examen fiable, précis et reproductible, notamment pour la fabrication sur mesure et personnalisée de bas ou de manchons.

La présente invention concerne également un ensemble comprenant le dispositif de mesure selon l'invention et un appareil apte à communiquer avec lesdits moyens de communication dudit dispositif de mesure. L'avantage de cet ensemble est de pouvoir disposer, sur un appareil apte à communiquer avec lesdits moyen de communication, de la mesure de circonférence sans avoir à retranscrire la mesure et ainsi d'éviter les erreurs de lecture et de saisie.

De préférence, l'ensemble selon l'invention est caractérisé en ce que :
- ledit appareil comprend une mémoire comprenant des données permettant de définir une position sur l'élément longitudinal, en ce que
- ledit appareil est apte à communiquer avec lesdits moyens de communication dudit dispositif de mesure pour transmettre lesdites données permettant de définir une position sur l'élément longitudinal, et en ce que
- lesdits moyens d'affichage sont aptes à afficher lesdites données pour indiquer à un utilisateur une ou plusieurs positions sur l'élément longitudinal.

L'avantage de ce mode de réalisation est que le dispositif de mesure, par l'intermédiaire des moyens d'affichage, permet d'indiquer à l'opérateur qui prend la mesure l'emplacement auquel il doit effectuer la mesure. Cela permet une prise de mesure plus rapide. Cela permet également d'effectuer plusieurs mesures sur un même objet ou un même membre corporel à des intervalles qui sont indiqués par l'appareil aux moyens d'affichage du dispositif de mesure et qui sont donc transmis à l'opérateur. L'opérateur dispose ainsi de toutes les informations concernant la mesure qu'il a à effectuer. Cela permet une meilleure cadence de prise de mesure ainsi qu'une meilleure fiabilité de la prise de mesure. La prise de mesure ainsi que le défilement de l'endroit où prendre la mesure sont contrôlés grâce aux moyens de contrôle.

De préférence, selon le dispositif de mesure suivant l'invention, l'élément longitudinal et/ou l'élément de mesure est enroulable. Par exemple, l'élément longitudinal et/ou l'élément de mesure peut se présenter sous forme d'un mètre ruban tels que ceux utilisés dans le domaine de la couture et qui sont généralement formés dans un matériau en plastique souple ou dans du papier présentant une forte résistance à la traction, ce qui permet d'enrouler ces mètres rubans soit manuellement soit automatiquement à l'aide d'un système d'enroulement automatique comprenant par exemple un ressort de rappel. Un enroulement l'élément longitudinal et/ou l'élément de mesure a pour avantage de minimiser la taille du dispositif de mesure lorsqu'il n'est pas utilisé, ce qui permet de le ranger sous une forme peu encombrante et de le transporter aisément. En outre, le fait que l'élément longitudinal et/ou l'élément de mesure puisse être enroulée permet à l'opérateur de n'en dérouler que la portion dont il a réellement besoin lors de la prise d'une mesure, la portion non utilisée de l'élément longitudinal et/ou l'élément de mesure restant enroulée, étant peu encombrante et n'entravant pas les mouvements de l'opérateur.

Avantageusement, le dispositif de mesure suivant l'invention comprend une pluralité de zones fixes de connexion. Selon l'invention un nombre illimité de zones fixes de connexion peuvent être présentes sur l'élément longitudinal. Lorsque plusieurs zones fixes de connexion sont présentes sur l'élément longitudinal, l'opérateur ou plusieurs opérateurs peuvent effectuer plusieurs mesures simultanément, ce qui permet de gagner du temps, notamment lorsqu'il convient d'effectuer des mesures ponctuelles selon un intervalle de quelques centimètres sur un segment corporel présentant une longueur relativement importante, par exemple une jambe.

De préférence, selon le dispositif de mesure suivant l'invention, l'élément longitudinal et/ou l'élément de mesure est en un matériau souple comme par exemple du plastique souple ou du papier présentant une résistance suffisante à la traction.

De préférence, suivant l'invention, l'élément longitudinal et/ou l'élément de mesure et/ou lesdites sangles distales et/ou proximales sont réalisées dans un matériau de type toile plastifiée ou en un matériau souple, par exemple un matériau en silicone ou en plastique souple.

Avantageusement, selon le dispositif de mesure suivant l'invention, ladite sangle distale et/ou proximale est détachable. Par exemple, ladite sangle distale et/ou proximale peut comprendre un moyen de fixation sous forme d'un organe mâle d'un système de fixation sur lequel se fixe un organe femelle situé sur l'élément longitudinal. Il pourrait également s'agir d'un moyen de fixation de type Velcro^{®} ou de tout autre type de moyen de fixation adéquat. Pouvoir détacher l'élément de mesure et/ou lesdites sangles distales et/ou proximales de l'élément longitudinal permet de minimiser la taille du dispositif de mesure lorsqu'il n'est pas utilisé mais aussi de changer d'élément de mesure si, par exemple, cet élément de mesure doit présenter une longueur ou une largeur moindre ou plus importante, ce qui est fonction du membre corporel considéré.

Par exemple, selon l'invention, ladite sangle proximale et/ou ladite sangle distale est un collier de type Colson^{®} réutilisable, c'est-à-dire qui peut être fermé puis ouvert. Il pourrait également s'agir d'un collier de type Colson^{®} à usage unique, c'est-à-dire uniquement prévu pour être fermé. Dans ce cas, si le collier est à usage unique, il conviendra de le couper afin de détacher le dispositif de mesure placé autour de l'objet dont la circonférence a été mesurée.

De façon avantageuse, selon le dispositif de mesure suivant l'invention, ladite sangle distale et/ou ladite sangle proximale est une bande longitudinale graduée ou non présentant une première et une deuxième extrémité munies chacune d'un moyen de fermeture et présentant une position ouverte et une position fermée, ladite position ouverte étant une position où lesdites première et deuxième extrémités sont espacées l'une de l'autre alors que ladite position fermée est une position dans laquelle ladite première extrémité de ladite bande longitudinale est rabattue sur ladite deuxième extrémité.

Avantageusement, selon l'invention, la sangle distale est une sangle présentant à la fois des systèmes d'attache mâles et femelles placés alternativement le long de ladite sangle distale pour faciliter tout attachement, ceci peu importe la forme de l'objet dont la circonférence est mesurée, en particulier peu importe la forme anatomique du membre corporel dont la circonférence est mesurée. Par exemple, ceci est intéressant au niveau de la cheville où un double attachement par croisement peu ainsi être réalisé de telle sorte que cet attachement de la sangle distale est sûr et se maintient en place pour permettre la réalisation de mesures précises et fiables quel que soit l'opérateur effectuant la mesure de la circonférence.

De préférence, selon le dispositif de mesure suivant l'invention, ledit moyen de fermeture est choisi dans le groupe constitué d'un système mâle-femelle, d'un système aimanté, d'une fermeture à bouton-pression, à bouton-poussoir ou par moyen auto-agrippant, par exemple de type Velcro^{®}. Un tel moyen de fermeture est particulièrement pratique puisqu'il peut être ouvert et fermé rapidement d'un simple mouvement de traction ou de pression. Il est bien entendu que tout autre type de moyen de fermeture adéquat fait également l'objet de la présente invention.

Eventuellement, selon l'invention, ladite sangle distale et/ou proximale est montée sur un coulisseau qui peut coulisser le long de l'élément longitudinal.

Selon l'invention, l'élément longitudinal souple, par exemple associé à un enrouleur, peut être relié/connecté à une sangle, par exemple à une sangle proximale, par l'intermédiaire d'un élément de connexion présentant une base dont la courbure permet d'épouser la surface de l'objet dont la circonférence doit être mesurée, par exemple la surface d'un membre corporel. Cette base peut présenter des fentes au travers desquelles passe la sangle (par exemple la sangle proximale) afin d'y être reliée. A la base peut être associé un plateau au départ duquel s'étendent des parois entre lesquelles l'enrouleur peut être inséré, en particulier lorsque l'élément longitudinal souple est associé à un enrouleur. Le plateau peut comprendre un organe mâle ou femelle coopérant avec un organe mâle ou femelle correspondant situé au niveau de l'élément longitudinal souple et/de l'enrouleur de telle sorte que ce dernier puisse être relié au plateau. Eventuellement, le plateau est rotatif, ce qui permet d'orienter l'élément longitudinal souple et/ou l'enrouleur, facilitant ainsi l'alignement de l'élément longitudinal souple avec l'objet ou le membre corporel dont la circonférence doit être mesurée et facilitant le rangement d'un dispositif selon l'invention dans une boite. Bien entendu, selon l'invention, la présence d'un enrouleur n'est pas indispensable et l'élément longitudinal souple pourrait être directement relié au plateau voire directement sur la base de l'élément de connexion si un plateau n'est pas présent. La présence d'un plateau n'est pas indispensable, l'élément longitudinal souple et/ou l'enrouleur pouvant être directement relié/connecté à l'élément de connexion.

D'autres formes de réalisation du dispositif de mesure de la circonférence de segments corporels suivant l'invention sont indiquées dans les revendications annexées.

L'invention a aussi pour objet un kit pour la mesure de la circonférence d'un objet, en particulier d'un membre corporel, comprenant :
- un élément longitudinal souple gradué ou non présentant une première direction longitudinale, agencé pour être apposé le long dudit objet, en particulier le long dudit membre corporel, ledit élément longitudinal souple étant relié à une sangle distale détachable ou non et/ou à une sangle proximale détachable ou non inscrite chacune dans un plan sensiblement perpendiculaire à ladite direction longitudinale, ledit élément longitudinal souple comprenant différentes zones fixes de connexion, et
- un système de mesure sous la forme d'un enrouleur d'un élément de mesure souple gradué ou non, agencé pour être apposé et former une boucle autour dudit objet, en particulier autour dudit membre corporel, lorsque ledit élément de mesure est en position de mesure, ledit système de mesure comprenant :
   - au moins une paroi et un orifice de sortie agencé pour permettre une sortie d'au moins une partie déroulée dudit élément de mesure, ladite au moins une partie déroulée dudit élément de mesure étant définie entre ledit orifice de sortie et une extrémité distale dudit élément de mesure, ladite extrémité distale dudit élément de mesure étant munie d'un élément de connexion, et
   - un moyen de connexion dudit l'élément de connexion de ladite extrémité distale dudit élément de mesure,
   - un organe de connexion, permettant d'assurer une connexion dudit système de mesure à au moins une des différentes zones fixes de connexion dudit élément longitudinal souple, ledit organe de connexion étant situé sur une paroi dudit système de mesure entre ledit orifice de sortie et ledit moyen de connexion dudit élément de connexion de ladite extrémité distale dudit élément de mesure.

Un tel kit pour la mesure de la circonférence de membres corporels comprend peu d'éléments qui peuvent être facilement et rapidement assemblés afin de disposer du dispositif de mesure de la circonférence de membres corporels tel que décrit ci-avant. Ce kit contenant tous les éléments essentiels de la présente invention.

D'autres formes de réalisation du kit pour la mesure de la circonférence de segments corporels suivant l'invention sont indiquées dans les revendications annexées.

D'autres caractéristiques, détails et avantages de l'invention ressortiront de la description donnée ci-après, à titre non limitatif et en faisant référence aux dessins annexés.

La présente invention porte encore sur une utilisation d'un dispositif de mesure selon l'invention et sur l'utilisation d'un kit selon l'invention pour mesurer la circonférence d'un objet, en particulier pour mesurer la circonférence d'un membre corporel.
La figure 1 illustre un exemple d'un dispositif de mesure de la circonférence selon l'invention sous sa forme montée pour la mesure de la circonférence d'un objet, en particulier pour la mesure de la circonférence d'un membre corporel.
Les figures 2A, 2B et 2C illustrent un exemple d'un système de mesure d'un dispositif de mesure selon l'invention pour la mesure de la circonférence d'un objet, en particulier pour la mesure de la circonférence d'un membre corporel.
La figure 3 illustre un autre mode de réalisation d'un dispositif de mesure de la circonférence selon l'invention sous sa forme montée pour la mesure de la circonférence d'un objet, en particulier pour la mesure de la circonférence d'un membre corporel.
Les figures 4A, 4B et 4C illustrent un autre mode de réalisation d'un système de mesure d'un dispositif de mesure selon l'invention pour la mesure de la circonférence d'un objet, en particulier pour la mesure de la circonférence d'un membre corporel.La figure 5 illustre un autre mode de réalisation d'un système de mesure d'un dispositif de mesure selon l'invention pour la mesure de la circonférence d'un objet, en particulier pour la mesure de la circonférence d'un membre corporel.
La figure 6 illustre un autre mode de réalisation d'un dispositif de mesure de la circonférence d'un objet selon l'invention sous sa forme montée pour la mesure de la circonférence d'un objet, en particulier pour la mesure de la circonférence d'un membre corporel.

Sur les figures, les éléments identiques ou analogues portent les mêmes références.

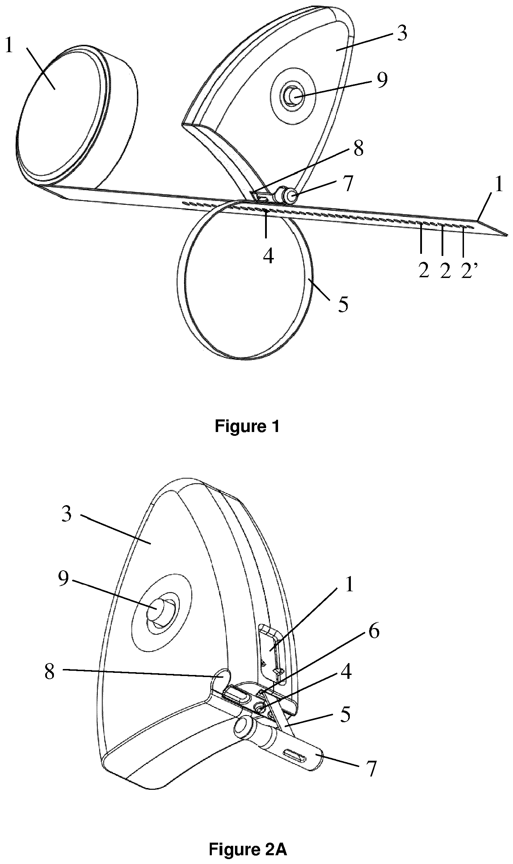

La figure 1 illustre un dispositif de mesure de la circonférence selon l'invention sous sa forme montée pour la mesure de la circonférence d'un objet, en particulier pour la mesure de la circonférence d'un membre corporel. Ce dispositif de mesure comprend un enrouleur 10 d'un élément longitudinal 1 souple présentant une première direction longitudinale et étant agencé pour être apposé le long de l'objet, en particulier le long du membre corporel. Cet élément longitudinal 1 souple est relié à une sangle distale et à une sangle proximale (non illustrées). Par ailleurs, cet élément longitudinal 1 souple comprend différentes zones fixes de connexion 2, 2', 2" sous forme de perforations situées à intervalles réguliers.

En outre, tel qu'illustré, le dispositif de mesure suivant l'invention comprend un système de mesure 3 comprenant un organe de connexion 4 sous forme d'une saillie assurant une connexion du système de mesure 3 à au moins une des différentes zones fixes de connexion 2, 2', 2" de l'élément longitudinal 1 souple. Cet organe de connexion 4, permettant d'assurer une connexion du système de mesure 3 sous la forme d'un enrouleur à au moins une des différentes zones fixes de connexion 2, 2', 2" de l'élément longitudinal 1 souple, est situé sur une paroi du système de mesure 3 entre l'orifice de sortie 6 et le moyen de connexion 8 de l'élément de connexion 7 de l'extrémité distale de l'élément de mesure 5.

Selon le mode de réalisation illustré, le système de mesure 3 est sous la forme d'un enrouleur d'un élément de mesure 5 souple agencé pour être apposé et former une boucle autour de l'objet, en particulier autour du membre corporel, lorsque l'élément de mesure 5 est en position de mesure. Comme illustré, le système de mesure 3 présente un orifice de sortie 6 agencé pour permettre une sortie d'au moins une partie déroulée de l'élément de mesure 5, l'extrémité distale de l'élément de mesure 5 étant munie d'un élément de connexion 7 sous la forme d'un cylindre. Le système de mesure 3 comprend également un moyen de connexion 8 de l'élément de connexion 7 de l'extrémité distale de l'élément de mesure 5, ce qui permet de former et de maintenir une boucle autour de l'objet, en particulier autour du membre corporel, dont la circonférence doit être mesurée.

En pratique, lors de la prise d'une mesure de circonférence d'un membre corporel, un opérateur place d'abord l'élément longitudinal 1 le long de ce membre corporel. Pour se faire, de préférence, l'élément longitudinal 1 est fixé/relié au membre corporel à l'aide d'une sangle distale et/ou d'une sangle proximale (non illustrées), la sangle distale et/ou la sangle proximale étant préférentiellement positionnée au niveau d'un repère anatomique fixe (zone articulaire ou osseuse) assurant que la sangle distale et/ou la sangle proximale soit bloquée au moins dans une direction. Ensuite, l'opérateur connecte le système de mesure 3 par insertion de l'organe de connexion 4 sous forme d'une saillie dans une des différentes zones fixe de connexion 2, 2', 2" de l'élément longitudinal 1. Le choix de la zone fixe de fixation 2, 2', 2" est effectué selon l'endroit le long du membre corporel où la mesure de circonférence doit être effectuée. Suite à l'insertion/la connexion de l'organe de connexion 4 sous forme d'une saillie dans une des différentes zones fixe de connexion 2, 2', 2", le système de mesure 3 est placé de façon fixe sur l'élément longitudinal 1 et l'opérateur peut alors dérouler l'élément de mesure 5 au départ du système de mesure 3 sous forme d'un enrouleur de telle sorte à former une boucle autour du membre corporel. Par insertion et connexion de l'élément de connexion 7 sous la forme d'un cylindre dans le moyen de connexion 8, la boucle est maintenue autour du membre corporel et une mesure de la circonférence de ce dernier peut être effectuée.

Selon le mode de réalisation illustré, le système de mesure est muni d'un moyen de commande 9 d'un système de blocage et de rappel de l'élément de mesure 5. Comme indiqué plus haut, ceci permet d'assurer un positionnement aisé de l'élément de mesure 5 par blocage d'une partie déroulée de ce dernier avec d'effectuer son rappel de telle sorte qu'il épouse étroitement et par contact le membre corporel dont la circonférence doit être mesurée.

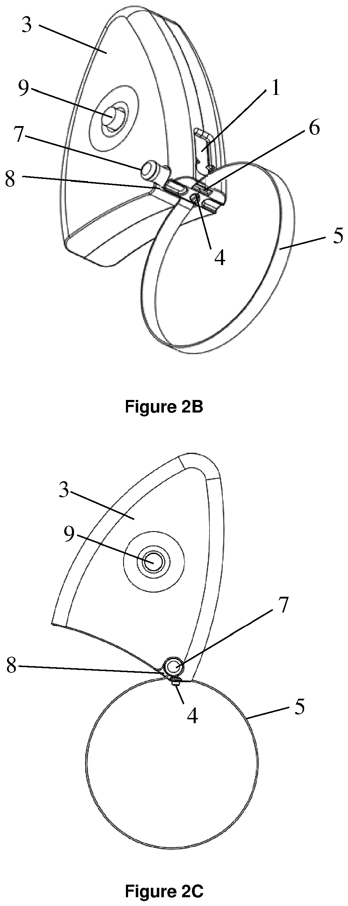

Les figures 2A, 2B et 2C illustrent le système de mesure d'un dispositif de mesure selon l'invention pour la mesure de la circonférence d'un objet, en particulier pour la mesure de la circonférence d'un membre corporel. La figure 2 A illustre le déroulement d'une partie de l'élément de mesure 5 tandis que les figures 2B et 2C illustrent la formation d'une boucle avec l'élément de mesure 5, l'organe de connexion 4 étant situé à l'intérieur de la boucle. Tel qu'illustré, le système de mesure 3 comprend une fenêtre de lecture 11 permettant de lire une valeur de la circonférence mesurée.

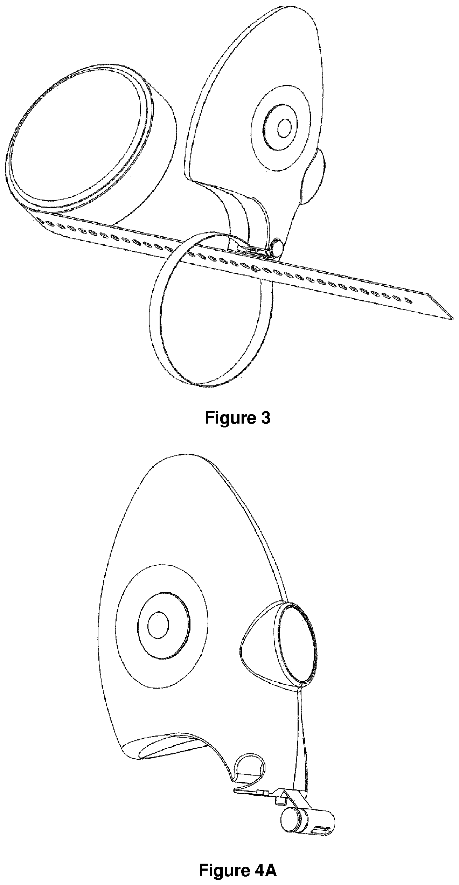

La figure 3 illustre un autre mode de réalisation d'un dispositif de mesure de la circonférence selon l'invention sous sa forme montée pour la mesure de la circonférence d'un objet, en particulier pour la mesure de la circonférence d'un membre corporel. Ce mode de réalisation est identique à celui illustré à la figure 1 à l'exception de la forme du système de mesure qui présente une excroissance ou saillie plus prononcée à l'extrémité de laquelle se retrouvent le moyen de connexion 8 de l'élément de connexion 7, l'orifice de sortie 6 agencé pour permettre une sortie d'au moins une partie déroulée de l'élément de mesure 5 et l'organe de connexion 4. Une telle configuration est préférée pour la réalisation de mesure de la circonférence d'objets ou de membres corporels fins (par exemple un doigt, aussi petite en soit la circonférence), c'est-à-dire pour la réalisation de mesure de la circonférence d'objets ou de membres corporels présentant une faible circonférence, ceci sans exclure une prise de mesure de circonférences plus importantes. En d'autres termes, une telle configuration d'un système de mesure rend le dispositif de mesure selon l'invention polyvalent en garantissant des prises de mesures précises et fiables quelle que soit la circonférence mesurée.

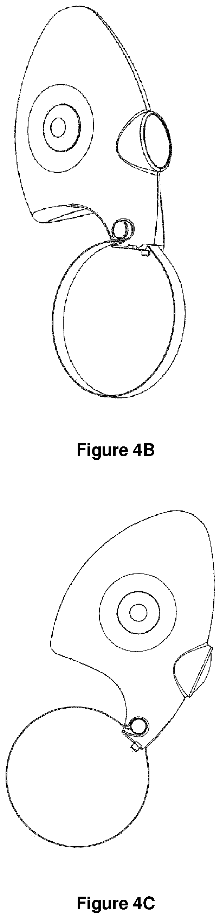

Les figures 4A, 4B et 4C illustrent un autre mode de réalisation d'un système de mesure d'un dispositif de mesure selon l'invention pour la mesure de la circonférence d'un objet, en particulier pour la mesure de la circonférence d'un membre corporel. Ces modes de réalisation sont identiques à ceux illustrés aux figures 2A, 2B et 2C à l'exception de la forme du système de mesure qui présente une excroissance ou saillie plus prononcée à l'extrémité de laquelle se retrouvent le moyen de connexion 8 de l'élément de connexion 7, l'orifice de sortie 6 agencé pour permettre une sortie d'au moins une partie déroulée de l'élément de mesure 5 et l'organe de connexion 4. Une telle configuration est préférée pour la réalisation de mesure de la circonférence d'objets ou de membres corporels fins, c'est-à-dire pour la réalisation de mesure de la circonférence d'objets ou de membres corporels présentant une faible circonférence, ceci sans exclure une prise de mesure de circonférences plus importantes. En d'autres termes, une telle configuration d'un système de mesure rend le dispositif de mesure selon l'invention polyvalent en garantissant des prises de mesures précises et fiables quelle que soit la circonférence mesurée.

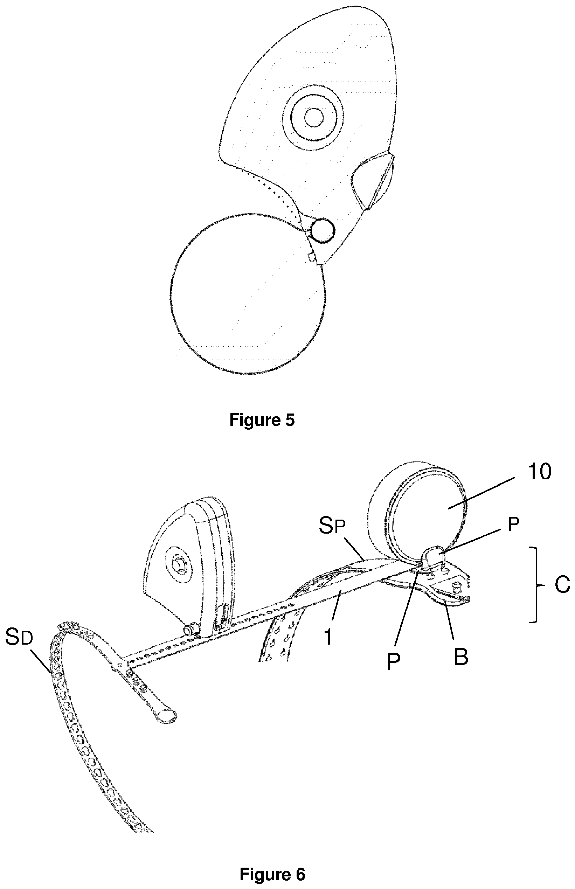

La figure 5 illustre un autre mode de réalisation d'un système de mesure d'un dispositif de mesure selon l'invention pour la mesure de la circonférence d'un objet, en particulier pour la mesure de la circonférence d'un membre corporel. Une telle configuration est plus préférée encore pour la réalisation de mesure de la circonférence d'objets ou de membres corporels fins, c'est-à-dire pour la réalisation de mesure de la circonférence d'objets ou de membres corporels présentant une faible circonférence, ceci sans exclure une prise de mesure de circonférences plus importantes. En d'autres termes, une telle configuration d'un système de mesure rend le dispositif de mesure selon l'invention polyvalent en garantissant des prises de mesures précises et fiables quelle que soit la circonférence mesurée.

La figure 6 illustre un mode de réalisation d'un dispositif de mesure de la circonférence selon l'invention sous sa forme montée. Les mêmes éléments que ceux illustrés aux figures 1 et 3 sont repris mais l'enrouleur 10 de l'élément longitudinal souple 1 est relié/connecté à une sangle proximale Sp par l'intermédiaire d'un élément de connexion C. Selon le mode de réalisation illustré, cet élément de connexion C présente une base B dont la courbure permet d'épouser la surface de l'objet dont la circonférence doit être mesurée, par exemple la surface d'un membre corporel. Cette base B présente des fentes F au travers desquelles passe la sangle proximale afin d'y être relié. Selon le mode de réalisation illustré, à la base B est associé un plateau PL au départ duquel s'étendent des parois p entre lesquelles l'enrouleur 10 peut être inséré. Le plateau PL peut comprendre un organe mâle ou femelle coopérant avec un organe mâle ou femelle correspondant situé au niveau de l'enrouleur 10 de telle sorte que ce dernier puisse être relié au plateau P. Eventuellement, le plateau P est rotatif. Comme illustré, le dispositif de mesure de la circonférence selon l'invention présente également une sangle distale Sd, laquelle comporte des éléments mâles (tenons) et femelles de connexion (trous).

## Revendications

1. Dispositif de mesure de la circonférence d'un objet, en particulier d'un membre corporel, comprenant :
- un élément longitudinal (1) souple gradué ou non présentant une première direction longitudinale, agencé pour être apposé le long dudit objet, en particulier le long dudit membre corporel, ledit élément longitudinal (1) souple étant relié à une sangle distale (S_{D}) détachable ou non et/ou à une sangle proximale (S_{P}) détachable ou non inscrite chacune dans un plan sensiblement perpendiculaire à ladite direction longitudinale, ledit élément longitudinal souple (1) comprenant différentes zones de connexion (2, 2', 2", ...), et
- un système de mesure (3) comprenant un organe de connexion (4) permettant d'assurer une connexion dudit système de mesure (3) à au moins une des différentes zones de connexion (2, 2', 2", ...) dudit élément longitudinal (1) souple, ledit système de mesure (3) étant sous la forme d'un enrouleur d'un élément de mesure (5) souple gradué ou non, agencé pour être apposé et former une boucle autour dudit objet, en particulier autour dudit membre corporel, lorsque ledit élément de mesure (5) est en position de mesure, ledit système de mesure (3) comprenant :
- au moins une paroi et un orifice de sortie (6) agencé pour permettre une sortie d'au moins une partie déroulée dudit élément de mesure (5), ladite au moins une partie déroulée dudit élément de mesure (5) étant définie entre ledit orifice de sortie (6) et une extrémité distale dudit élément de mesure (5), ladite extrémité distale dudit élément de mesure (5) étant munie d'un élément de connexion (7), et
- un moyen de connexion (8) dudit l'élément de connexion (7) de ladite extrémité distale dudit élément de mesure (5),
ledit organe de connexion (4), permettant d'assurer une connexion dudit système de mesure (3) sous la forme d'un enrouleur à au moins une des différentes zones de connexion (2, 2', 2", ...) dudit élément longitudinal (1) souple,
ledit dispositif de mesure étant **caractérisé en ce que** lesdites différentes zones de connexion (2, 2', 2", ...) dudit élément longitudinal souple (1) sont des zones fixes de connexion, et **en ce que** ledit organe de connexion (4) est situé sur une paroi dudit système de mesure (3) entre ledit orifice de sortie (6) et ledit moyen de connexion (8) dudit élément de connexion (7) de ladite extrémité distale dudit élément de mesure (5).

2. Dispositif de mesure selon la revendication 1, **caractérisé en ce que** ledit organe de connexion (4) du système de mesure (3) et/ou lesdites différentes zones fixes de connexion (2, 2', 2", ...) dudit élément longitudinal (1) est/sont configuré(s) de telle sorte qu'il(s) assure(nt) une inscription et un maintien dudit élément de mesure (5) dans un plan perpendiculaire à ladite direction longitudinale dudit élément longitudinal (1).

3. Dispositif de mesure selon la revendication 1 ou 2, **caractérisé en ce que** ledit organe de connexion (4) dudit système de mesure (3) est un organe de type mâle ou femelle agencé pour coopérer et assurer une connexion avec au moins une desdites zones fixes de connexion (2, 2', 2", ...) dudit élément longitudinal (1) étant une zone de type mâle ou femelle correspondante.

4. Dispositif de mesure selon l'une quelconque des revendications précédentes, **caractérisé en ce que** ladite boucle formée autour dudit objet, en particulier autour dudit membre corporel, est inscrite dans un seul et unique plan passant par un axe longitudinal médian défini le long dudit élément de mesure (5).

5. Dispositif de mesure selon l'une quelconque des revendications précédentes, **caractérisé en ce que** ledit système de mesure (3) comprend un système de rappel dudit élément de mesure, par exemple un ressort ou un moteur, exerçant sur ledit élément de mesure (5) une tension liée à un poids compris entre 5 et 5000 g, préférentiellement entre 5 et 3000 g, préférentiellement entre 15 et 1500 g, plus préférentiellement encore entre 15 et 1000 g, avantageusement entre 15 et 500 g, plus avantageusement entre 15 et 150 g.

6. Dispositif de mesure selon l'une quelconque des revendications précédentes, **caractérisé en ce qu'**il comprend en outre :
- un module électronique de mesure apte à déterminer une mesure de circonférence à partir d'un déroulement dudit élément de mesure (5) ; et
- des moyens d'affichages pour afficher une mesure de circonférence déterminée par ledit module électronique.

7. Dispositif de mesure selon l'une quelconque des revendications précédentes, **caractérisé en ce qu'**il comprend en outre des moyens de communication pour communiquer à un autre dispositif électronique une mesure de circonférence déterminée par ledit module électronique.

8. Kit pour former un dispositif de mesure selon l'une quelconque des revendications 1 à 7, comprenant :
- un élément longitudinal (1) souple gradué ou non présentant une première direction longitudinale, agencé pour être apposé le long dudit objet, en particulier le long dudit membre corporel, ledit élément longitudinal (1) souple étant relié à une sangle distale détachable ou non et/ou à une sangle proximale détachable ou non inscrite chacune dans un plan sensiblement perpendiculaire à ladite direction longitudinale, ledit élément longitudinal (1) souple comprenant différentes zones fixes de connexion (2, 2', 2", ...), et
- un système de mesure (3) sous la forme d'un enrouleur d'un élément de mesure (5) souple gradué ou non, agencé pour être apposé et former une boucle autour dudit objet, en particulier autour dudit membre corporel, lorsque ledit élément de mesure (5) est en position de mesure, ledit système de mesure (3) comprenant :
- au moins une paroi et un orifice de sortie (6) agencé pour permettre une sortie d'au moins une partie déroulée dudit élément de mesure (5), ladite au moins une partie déroulée dudit élément de mesure (5) étant définie entre ledit orifice de sortie (6) et une extrémité distale dudit élément de mesure (5), ladite extrémité distale dudit élément de mesure (5) étant munie d'un élément de connexion (7), et
- un moyen de connexion (8) dudit l'élément de connexion (7) de ladite extrémité distale dudit élément de mesure (5),
- un organe de connexion (4), permettant d'assurer une connexion dudit système de mesure (3) à au moins une des différentes zones fixes de connexion (2, 2', 2", ...) dudit élément longitudinal (1) souple, ledit organe de connexion (4) étant situé sur une paroi dudit système de mesure (3) entre ledit orifice de sortie (6) et ledit moyen de connexion (8) dudit élément de connexion (7) de ladite extrémité distale dudit élément de mesure (5).

9. Utilisation d'un dispositif de mesure selon l'une quelconque des revendications 1 à 7 ou d'un kit selon la revendication 8 pour mesurer la circonférence d'un objet, en particulier pour mesurer la circonférence d'un membre corporel.

## Patentansprüche

1. Vorrichtung zur Messung des Umfangs eines Objekts, insbesondere eines Körperglieds, umfassend:
- ein flexibles, graduiertes oder nicht graduiertes längliches Element (1), das eine erste Längsrichtung aufweist, das angeordnet ist, um entlang des Objekts, insbesondere entlang des Körperglieds, angebracht zu werden, wobei das flexible längliche Element (1) mit einem abnehmbaren oder nicht abnehmbaren distalen Gurt (S_{D}) und/oder mit einem abnehmbaren oder nicht abnehmbaren proximalen Gurt (S_{P}) verbunden ist, die jeweils in einer Ebene im Wesentlichen senkrecht zu der Längsrichtung aufgenommen sind, wobei das flexible längliche Element (1) verschiedene Verbindungsbereiche (2, 2', 2", ...) umfasst, und
- ein Messsystem (3), das ein Verbindungsorgan (4) umfasst, das es ermöglicht, eine Verbindung des Messsystems (3) mit mindestens einem der verschiedenen Verbindungsbereiche (2, 2', 2", ...) des flexiblen länglichen Elements (1) sicherzustellen, wobei das Messsystem (3) in Form einer Aufwicklung eines flexiblen, graduierten oder nicht graduierten Messelements (5) vorliegt, das angeordnet ist, um angebracht zu werden und eine Schlaufe um das Objekt, insbesondere um das Körperglied, zu bilden, wenn sich das Messelement (5) in der Messposition befindet, wobei das Messsystem (3) Folgendes umfasst:
- mindestens eine Wand und eine Austrittsöffnung (6), die angeordnet ist, um einen Austritt von mindestens einem abgewickelten Teil des Messelements (5) zu ermöglichen, wobei der mindestens eine abgewickelte Teil des Messelements (5) zwischen der Austrittsöffnung (6) und einem distalen Ende des Messelements (5) definiert ist, wobei das distale Ende des Messelements (5) mit einem Verbindungselement (7) versehen ist, und
- ein Verbindungsmittel (8) des Verbindungselements (7) des distalen Endes des Messelements (5),
wobei das Verbindungsorgan (4) das Sicherstellen einer Verbindung des Messsystems (3) in Form einer Aufwicklung mit mindestens einem der verschiedenen Verbindungsbereiche (2, 2', 2", ...) des flexiblen länglichen Elements (1) ermöglicht,
wobei die Messvorrichtung **dadurch gekennzeichnet ist, dass** die verschiedenen Verbindungsbereiche (2, 2', 2", ...) des flexiblen länglichen Elements (1) feste Verbindungsbereiche sind, und dass das Verbindungsorgan (4) sich an einer Wand des Messsystems (3) zwischen der Austrittsöffnung (6) und dem Verbindungsmittel (8) des Verbindungselements (7) des distalen Endes des Messelements (5) befindet.

2. Messvorrichtung nach Anspruch 1, **dadurch gekennzeichnet, dass** das Verbindungsorgan (4) des Messsystems (3) und/oder die verschiedenen festen Verbindungsbereiche (2, 2', 2", ...) des länglichen Elements (1) derart konfiguriert ist/sind, dass es/sie ein Aufnehmen und ein Halten des Messelements (5) in einer Ebene senkrecht zur Längsrichtung des länglichen Elements (1) sicherstellt/sicherstellen.

3. Messvorrichtung nach Anspruch 1 oder 2, **dadurch gekennzeichnet, dass** das Verbindungsorgan (4) des Messsystems (3) ein Organ vom männlichen oder weiblichen Typ ist, das angeordnet ist, um mit mindestens einem der festen Verbindungsbereiche (2, 2', 2", ...) des länglichen Elements (1), das ein entsprechender Bereich vom männlichen oder weiblichen Typ ist, zusammenzuwirken und eine Verbindung mit diesem sicherzustellen.

4. Messvorrichtung nach einem der vorstehenden Ansprüche, **dadurch gekennzeichnet, dass** die um das Objekt, insbesondere um das Körperglied, gebildete Schlaufe in ein und derselben Ebene aufgenommen ist, die durch eine Längsmittelachse verläuft, die entlang des Messelements (5) definiert ist.

5. Messvorrichtung nach einem der vorstehenden Ansprüche, **dadurch gekennzeichnet, dass** das Messsystem (3) ein System zur Rückstellung des Messelements umfasst, zum Beispiel eine Feder oder einen Motor, das auf das Messelement (5) eine Spannung ausübt, die mit einem Gewicht zwischen 5 und 5000 g, bevorzugt zwischen 5 und 3000 g, bevorzugt zwischen 15 und 1500 g, noch bevorzugter zwischen 15 und 1000 g, vorteilhafterweise zwischen 15 und 500 g, noch vorteilhafter zwischen 15 und 150 g verbunden ist.

6. Messvorrichtung nach einem der vorstehenden Ansprüche, **dadurch gekennzeichnet, dass** sie weiter Folgendes umfasst:
- ein elektronisches Messmodul, das in der Lage ist, eine Umfangsmessung ausgehend von einer Abwicklung des Messelements (5) zu bestimmen; und
- Anzeigemittel zum Anzeigen einer Umfangsmessung, die durch das elektronische Modul bestimmt wurde.

7. Messvorrichtung nach einem der vorstehenden Ansprüche, **dadurch gekennzeichnet, dass** sie weiter Kommunikationsmittel umfasst, um einer anderen elektronischen Vorrichtung eine durch das elektronische Modul bestimmte Umfangsmessung mitzuteilen.

8. Kit zum Bilden einer Messvorrichtung nach einem der Ansprüche 1 bis 7, umfassend:
- ein flexibles, graduiertes oder nicht graduiertes längliches Element (1), das eine erste Längsrichtung aufweist, das angeordnet ist, um entlang des Objekts, insbesondere entlang des Körperglieds, angebracht zu werden, wobei das flexible längliche Element (1) mit einem abnehmbaren oder nicht abnehmbaren distalen Gurt und/oder mit einem abnehmbaren oder nicht abnehmbaren proximalen Gurt verbunden ist, die jeweils in einer Ebene im Wesentlichen senkrecht zu der Längsrichtung aufgenommen sind, wobei das flexible längliche Element (1) verschiedene Verbindungsbereiche (2, 2', 2", ...) umfasst, und
- ein Messsystem (3) in Form einer Aufwicklung eines flexiblen, graduierten oder nicht graduierten Messelements (5), das angeordnet ist, um angebracht zu werden und eine Schlaufe um das Objekt, insbesondere um das Körperglied, zu bilden, wenn sich das Messelement (5) in der Messposition befindet, wobei das Messsystem (3) Folgendes umfasst:
- mindestens eine Wand und eine Austrittsöffnung (6), die angeordnet ist, um einen Austritt von mindestens einem abgewickelten Teil des Messelements (5) zu ermöglichen, wobei der mindestens eine abgewickelte Teil des Messelements (5) zwischen der Austrittsöffnung (6) und einem distalen Ende des Messelements (5) definiert ist, wobei das distale Ende des Messelements (5) mit einem Verbindungselement (7) versehen ist, und
- ein Verbindungsmittel (8) des Verbindungselements (7) des distalen Endes des Messelements (5),
- ein Verbindungsorgan (4), das eine Verbindung des Messsystems (3) mit mindestens einem der verschiedenen festen Verbindungsbereiche (2, 2', 2", ...) des flexiblen länglichen Elements (1) ermöglicht, wobei das Verbindungsorgan (4) sich an einer Wand des Messsystems (3) zwischen der Austrittsöffnung (6) und dem Verbindungsmittel (8) des Verbindungselements (7) des distalen Endes des Messelements (5) befindet.

9. Verwendung einer Messvorrichtung nach irgendeinem der Ansprüche 1 bis 7 oder eines Kits nach Anspruch 8 zum Messen des Umfangs eines Objekts, insbesondere zum Messen des Umfangs eines Körperglieds.

## Claims

1. A device for measuring the circumference of an object, in particular of a body limb, comprising:
- a flexible longitudinal element (1), graduated or not, having a first longitudinal direction, arranged to be affixed along said object, in particular along said body limb, said flexible longitudinal element (1) being connected to a detachable or not detachable distal strap (S_{D}) and/or to a detachable or not detachable proximal strap (S_{P}), each inscribed in a plane substantially perpendicular to said longitudinal direction, said flexible longitudinal element (1) comprising different connection zones (2, 2', 2",...) and
- a measuring system (3) comprising a connection member (4) to ensure a connection of said measuring system (3) to at least one of the different connection zones (2, 2', 2", ...) of said flexible longitudinal element (1), said measuring system (3) being in the form of a reel of a flexible measuring element (5), graded or not, arranged to be affixed and to form a loop around said object, in particular around said body limb, when said measuring element (5) is in the measuring position, said measuring system (3) comprising :
- at least one wall and an exit port (6) arranged to allow an exit of at least one unwound portion of said measuring element (5), said at least one unwound portion of said measuring element (5) being defined between said exit port (6) and a distal end of said measuring element (5), said distal end of said measuring element (5) being provided with a connecting element (7), and
- means for connecting (8) said connecting element (7) of said distal end of said measuring element (5),
said connection member (4), allowing to ensure a connection of said measuring system (3) in the form of a reel to at least one of the different connection zones (2, 2', 2", ...) of said flexible longitudinal element (1),
said measuring device being **characterized in that** said different connection zones (2, 2', 2", ...) of said flexible longitudinal element (1) are fixed zones of connection, and **in that** said connection member (4) is located on a wall of said measuring system (3) between said exit port (6) and said connection means (8) of said connecting element (7) of said distal end of said measuring element (5).

2. The device for measuring according to claim 1, **characterized in that** said connection member (4) of the measuring system (3) and/or said different fixed zones of connection (2, 2', 2", ...) of said longitudinal element (1) is/are configured such that it/they ensure(s) an inscription and a holding of said measuring element (5) in a plane perpendicular to said longitudinal direction of said longitudinal element (1).

3. The device for measuring according to claim 1 or 2, **characterized in that** said connection member (4) of said measuring system (3) is a male or female type member arranged to cooperate and ensure a connection with at least one of said fixed zones of connection (2, 2', 2", ...) of said longitudinal element (1) being a corresponding male or female type zone.

4. The device for measuring according to any one of the preceding claims, **characterized in that** said loop formed around said object, in particular around said body limb, is inscribed in one and only one plane passing through a median longitudinal axis defined along said measuring element (5).

5. The device for measuring according to any one of the preceding claims, **characterized in that** said measuring system (3) comprises a system for returning said measuring element, for example a spring or a motor, exerting on said measuring element (5) a tension related to a weight between 5 and 5000 g, preferably between 5 and 3000 g, preferably between 15 and 1500 g, even more preferably between 15 and 1000 g, advantageously between 15 and 500 g, more advantageously between 15 and 150 g.

6. The device for measuring according to any one of the preceding claims, **characterised in that** it further comprises:
- an electronic measuring module capable of determining a circumference measurement from an unwinding of said measuring element (5); and
- display means for displaying a circumference measurement determined by said electronic module.

7. The device for measuring according to any one of the preceding claims, **characterized in that** it further comprises communication means for communicating a circumference measurement determined by said electronic module to another electronic device.

8. A kit for forming a measuring device according to any one of claims 1 to 7, comprising:
- a flexible longitudinal element (1) graduated or not having a first longitudinal direction, arranged to be affixed along said object, in particular along said body limb, said flexible longitudinal element (1) being connected to a detachable or not detachable distal strap and/or to a detachable or not detachable proximal strap each inscribed in a plane substantially perpendicular to said longitudinal direction, said flexible longitudinal element (1) comprising different fixed zones of connection (2, 2', 2",...), and
- a measuring system (3) in the form of a reel of a flexible measuring element (5), graduated or not, arranged to be affixed and form a loop around said object, in particular around said body limb, when said measuring element (5) is in measuring position, said measuring system (3) comprising:
- at least one wall and an exit port (6) arranged to allow an exit of at least one unwound portion of said measuring element (5), said at least one unwound portion of said measuring element (5) being defined between said exit port (6) and a distal end of said measuring element (5), said distal end of said measuring element (5) being provided with a connecting element (7), and
- means for connecting (8) said connecting element (7) of said distal end of said measuring element (5),
- a connection member (4), for providing a connection of said measuring system (3) to at least one of the different fixed zones of connection (2, 2', 2", ...) of said flexible longitudinal element (1), said connection member (4) being located on a wall of said measuring system (3) between said exit port (6) and said connection means (8) of said connecting element (7) of said distal end of said measuring element (5).

9. Use of a device for measuring according to any one of claims 1 to 7 or of a kit according to claim 8 for measuring the circumference of an object, in particular for measuring the circumference of a body limb.
